# EUROPÄISCHE PATENTSCHRIFT

(11) **EP 1 646 337 B1**
(45) Veröffentlichungstag und Bekanntmachung des Hinweises auf die Patenterteilung: **19.03.2008**
(21) Anmeldenummer: 04739950.6
(22) Anmeldetag: 16.06.2004
(51) Int. Cl.: A61F 2/44

(54) **BANDSCHEIBENPROTHESE**
INTERVERTEBRAL DISK PROSTHESIS
PROTHESE DE DISQUE INTERVERTEBRAL

(30) Priorität: 21.07.2003 EP 03016441
(43) Veröffentlichungstag der Anmeldung: 19.04.2006
(73) Patentinhaber: Cervitech, Inc., Rockaway, NJ 07866 (US)
(72) Erfinder: KELLER, Arnold, 23863 Kayhude (DE)
(74) Vertreter: Glawe, Delfs, Moll
(86) Internationale Anmeldenummer: PCT/EP2004/006484
(87) Internationale Veröffentlichungsnummer: WO 2005/018508

(56) Entgegenhaltungen:
- FR-A- 2 632 516
- FR-A- 2 718 635
- FR-A- 2 780 635

## Beschreibung

Ein bekannter Typ von Bandscheibenprothesen (WO 01/01893) weist zwei Deckplatten aus Metall und einen Prothesenkern aus Polyethylen auf, der mit einer der beiden Deckplatten ein Gleitflächenpaar bildet und an der anderen Deckplatte befestigt ist. Für die Befestigung sind an der Deckplatte Randleisten vorgesehen, deren hinterschnittenes Verbindungsprofil zusammenwirkt mit einem komplementären Verbindungsprofil an der Unterseite des Prothesenkerns. Am rechten und linken lateralen Rand der Deckplatte und des Prothesenkerns sind solche Verbindungsprofile parallel zueinander vorgesehen. Außerdem befindet sich ein solches, geradlinig gestrecktes Profil am dorsalen Rand der Deckplatte und des Prothesenkerns. Am ventralen Rand fehlt ein solches Profil, so daß der Prothesenkern von der ventralen Seite her in die Verbindungsprofile der Deckplatte eingeschoben werden kann. Durch ein Paar von am ventralen Rand vorgesehenen Rastelementen wird der Prothesenkern in der montierten Stellung festgehalten. Obwohl die Verbindungsprofile nahezu spielfrei hergestellt werden, besteht ein wichtiges Anliegen der Prothesenherstellung darin, auch im Falle von Herstellungstoleranzen ein Bewegungsspiel des Prothesenkerns gegenüber der ihn haltenden Deckplatte auszuschließen. Insbesondere sollen Rotationsbewegungen des Prothesenkerns gegenüber der ihn haltenden Deckplatte ausgeschlossen werden.

Der Stand der Technik ergibt keine Hinweise darauf, wie dieses Problem gelöst werden könnte. Die FR-A-2718635 zeigt Verbindungsprofile lediglich an parallelen, seitlichen Rändern der Deckplatte. Sie fehlen in sämtlichen nicht seitlichen und nicht parallelen Randbereichen. Für Knieprothesen zeigt die FR-A-2632516 die Befestigung eines Polyethylenteils in einem Halteprofil mittels hufeisenförmig umlaufender Verbindungsprofile, wobei jedoch keine Sicherheit dafür geschaffen ist, daß auch die nicht parallelen Abschnitte der Verbindungsprofile miteinander in Eingriff bleiben. Die FR-A-2780635 zeigt für eine Schulterprothese die Befestigung eines Polyethylenteils auf einem Halteteil mittels ausschließlich winklig zueinander verlaufender Verbindungsprofile. Mit den Formerfordernissen einer Bandscheibenprothese läßt sich dies nicht in Einklang bringen.

Die Erfindung hat erkannt, daß sich das Problem bei Bandscheibenprothesen durch die in Anspruch 1 angegebene besondere Anordnung der Verbindungsprofile lösen läßt. Demnach ist vorgesehen, daß die Verbindungsprofile mindestens ein Paar von zur AP-Richtung symmetrisch und zueinander winklig angeordneten Verbindungsprofilabschnitten umfassen. Unter der AP-Richtung ist die anterior-posteriore Richtung zu verstehen. Die erfindungsgemäß winklig zueinander angeordneten Profilabschnitte erzeugen eine Dreieckskonfiguration, die einer Verdrehung des Prothesenkerns gegenüber der Deckplatte entgegenwirkt, falls zwischen den Verbindungsprofilen der Deckplatte und des Prothesenkerns Spiel vorhanden ist. Die Verbindungsprofile können im wesentlichen ausschließlich von derartigen winklig zueinander angeordneten Profilabschnitten gebildet sein. Diese können aber auch zusätzlich zu parallel an den lateralen Rändern der Deckplatte und des Prothesenkerns angeordneten Verbindungsprofilen vorgesehen sein. Sie sollen im wesentlichen geradlinig gestreckt sein und sind vorzugsweise zumindest dorsal angeordnet. Der Winkel zwischen den winklig zueinander angeordneten Profilabschnitten soll 150° nicht überschreiten. Vorzugsweise liegt die Grenze etwa bei 140°.

Die Erfindung wird im folgenden näher unter Bezugnahme auf die Zeichnung erläutert, die ein vorteilhaftes Ausführungsbeispiel veranschaulicht. Darin zeigen:
- Fig. 1: eine perspektivische Ansicht der Endplatte mit daran befestigtem Prothesenkern,
- Fig. 2 bis 4: unterschiedliche Ansichten derselben Endplatte ohne Prothesenkern und
- Fig. 5 und 6: perspektivische Ansichten des Prothesenkerns.

Die Endplatte 1, die normalerweise als untere Endplatte eingesetzt wird, hat einen etwa ovalen Umriß und weist eine gezahnte Befestigungsfläche 2 zur Auflage auf den Wirbelkörper und einen Flansch 3 auf, der an der ventralen Seite des Wirbelkörpers zu liegen kommt und durch Schraubenlöcher 4 damit verschraubt werden kann. Auf der der Befestigungsfläche 2 gegenüber liegenden Seite, die normalerweise oben liegt, bildet die Deckplatte 1 ein Lager für den Prothesenkern 5. Dieses Lager besteht aus einer ebenen Fläche 6 und einem diese umgebenden Rand 7, der aus zwei parallelen Abschnitten 7' an den lateralen Seiten der Platte und einem dorsalen Rand 7" zusammengesetzt ist. Die Außenkontur des Randes 7 entspricht der ovalen Kontur der Deckplatte. Der Rand ist auf seiner der Fläche 6 zugewendeten Seite mit einer Profilleiste 8 und darunter mit einem Hinterschnitt 8' versehen. Das dadurch an der Innenseite des Rands 7 gebildete Verbindungsprofil verläuft in den lateralen Abschnitten 7' gerade und auf beiden Seiten parallel. Im dorsalen Abschnitt 7" setzt sich das Verbindungsprofil aus zwei Abschnitten 9 zusammen. In jedem dieser Abschnitte verläuft das Verbindungsprofil geradlinig. Die Profile beider Abschnitte sind symmetrisch zur AP-Achse 10 des Implantats angeordnet und schließen ein Winkel α ein, der im dargestellten Beispiel bei 140° liegt.

Die Oberseite des Rands 7 bildet eine ebene Fläche 6', die parallel zur Lagerfläche 6 verläuft und gleichfalls eine Lagerfläche für den Prothesenkern bildet.

Der Flansch 3 der Deckplatte enthält einen Schlitz 11, der lotrecht zur Verbindungsprofil 6 verläuft und dessen Funktion später erläutert wird.

Der Prothesenkern 5 bildet auf seiner Oberseite 12 eine Gleitfläche, die mit der zweiten, nicht dargestellten Deckplatte zur Bildung eines Gelenks zusammenwirkt. Der Umriß des Prothesenkerns stimmt mit demjenigen der Deckplatte 1 überein. Auf seiner Unterseite weist er eine ebene Fläche 13 auf, die zur Auflage auf der Lagerfläche 6 der Endplatte 1 bestimmt ist und im wesentlichen dieselbe Umrißgestalt hat. Sie wird begrenzt durch ein Verbindungsprofil, das von einer Profilleiste 14 und einem Hinterschnitt 14' gebildet ist. Dieses Verbindungsprofil stimmt mit dem Verbindungsprofil 8, 9 der Randleiste 7 der Deckplatte 1 komplementär überein. Insbesondere setzt sich das Verbindungsprofil aus zwei lateralen Abschnitten 15 und zwei dorsalen Abschnitten 16 zusammen, die jeweils den lateralen und dorsalen Abschnitten des Rands 7 der Deckplatte 1 entsprechen und mit diesen formflüssig zusammenwirken können.

Außerhalb des Verbindungsprofils 14, 14' befindet sich an der Unterseite des Prothesenkerns 5 eine ebene Fläche 17, die der Lagerfläche 6' auf der Oberseite des Rands 7 der Deckplatte 1 entspricht und tragend mit ihr zusammenwirkt, wenn der Prothesenkern an der Deckplatte 1 montiert ist.

Die Montage findet dadurch statt, daß der Prothesenkern mit seinem Verbindungsprofil 14, 14' in die entsprechenden Verbindungsprofile 8, 8' von der offenen, ventralen Seite her eingeschoben wird. Wenn der Prothesenkern vollständig eingeschoben ist, greift seine Profilleiste 14 nicht nur im Bereich der seitlichen Abschnitte 7', sondern auch des dorsalen Abschnitts 7" in den Hinterschnitt 8' des Rands 7 der Deckplatte ein, wobei die Verbindungsprofile im wesentlichen vollflächig aneinander liegen.

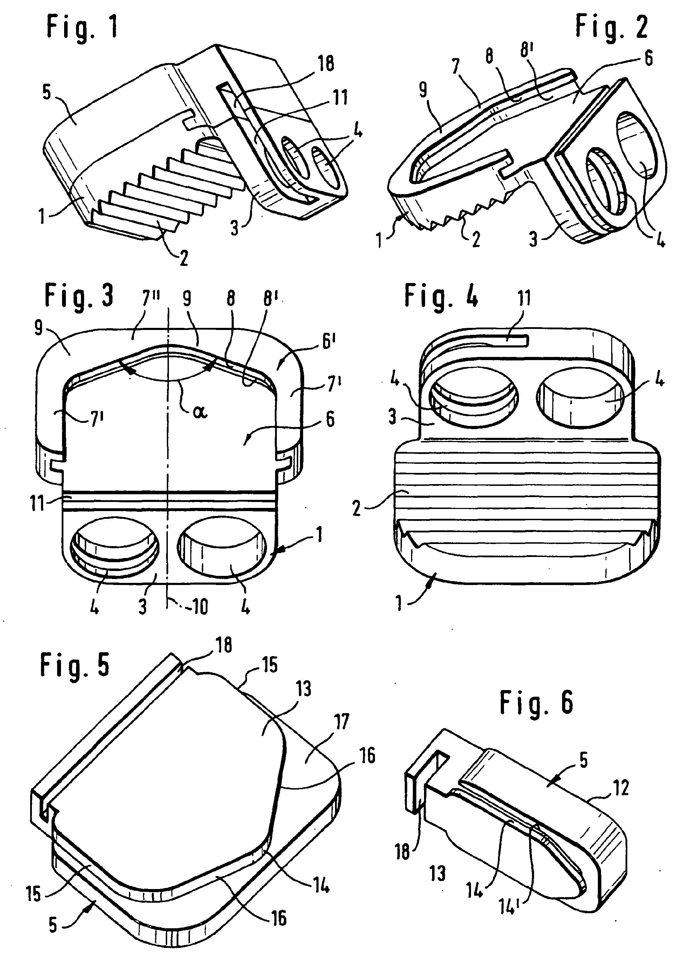

An derjenigen Stelle, an der sich der Schlitz 11 im Spalt 3 befindet, weist der Prothesenkern eine Nut 18 auf, die mit dem Spalt 11 fluchtet, wenn der Prothesenkern seine Endstellung erreicht hat, in welcher die dorsalen Verbindungsprofile aneinander anschlagen. Es wird eine nicht gezeigte Lamelle in den Spalt 11 und die Nut 18 eingeschoben, die den Prothesenkern in dieser Position gegenüber der Deckplatte 1 sichert. Die Lamelle ihrerseits wird dadurch gesichert, daß sie an der Stelle des in Fig. 3 links erscheinenden Schraubenlochs ebenfalls eine Bohrung aufweist, durch die eine etwaige Befestigungsschraube hindurchgeht.

Dadurch, daß die Verbindungsprofile des Prothesenkerns und der Deckplatte mit den sich zu einem Dreieck ergänzenden Abschnitten 9 bzw. 16 ausgestattet sind, wird im Vergleich mit bekannten Prothesen eine gesteigerte Lagesicherheit des Prothesenkerns gegenüber der Deckplatte 1 erzielt. Insbesondere wird seine Sicherheit gegenüber Relativbewegungen im Rotationssinne dadurch gesteigert.

## Patentansprüche

1. Bandscheibenprothese mit zwei Deckplatten und einem Prothesenkern (5), der mit einer der Deckplatten (1) durch komplementär hinterschnittene Verbindungsprofile (8, 8', 14, 14')verbunden ist, **dadurch gekennzeichnet, daß** die Verbindungsprofile (8, 8', 14, 14') mindestens ein Paar von zur AP-Richtung (10) symmetrisch und zueinander winklig angeordneten Profilabschnitten (9, 16) umfassen.

2. Bandscheibenprothese nach Anspruch 1, **dadurch gekennzeichnet, daß** die zueinander winklig angeordneten Profilabschnitte (9, 16) im wesentlichen geradlinig gestreckt sind.

3. Bandscheibenprothese nach Anspruch 1 oder 2, **dadurch gekennzeichnet, daß** die zueinander winklig angeordneten Profilabschnitte (9, 16) dorsal angeordnet und durch eine ventral angeordnete Einrichtung (11, 18) in Eingriff gehalten sind.

4. Bandscheibenprothese nach Anspruch 3, **dadurch gekennzeichnet, daß** die dorsal angeordneten Profilabschnitte(9, 16) zusätzlich zu einem Paar lateral angeordneter Profilabschnitte (7') vorgesehen sind.

5. Bandscheibenprothese nach Anspruch 1 oder 2, **dadurch gekennzeichnet, daß** der Winkel (α) zwischen den zueinander winklig angeordneten Profilabschnitten (9, 16) nicht größer als 150° ist.

## Claims

1. An intervertebral disc prosthesis with two cover plates and a prosthesis core (5) which is connected to one of the cover plates (1) by means of connection profiles (8, 8', 14, 14') which are undercut in a complementary manner, **characterized in that** the connection profiles (8, 8', 14, 14') include at least one pair of profile sections (9, 16) which are symmetrical with respect to the AP direction (10) and are arranged at an angle to one another.

2. The intervertebral disc prosthesis as claimed in claim 1, **characterized in that** the profile sections (9, 16) arranged at an angle to one another run in a substantially straight line.

3. The intervertebral disc prosthesis as claimed in claim 1 or 2, **characterized in that** the profile sections (9, 16) arranged at an angle to one another are arranged on the dorsal side and are held in engagement by means (11, 18) arranged on the ventral side.

4. The intervertebral disc prosthesis as claimed in claim 3, **characterized in that** the profile sections (9, 16) arranged on the dorsal side are provided in addition to a pair of laterally arranged profile sections (7').

5. The intervertebral disc prosthesis as claimed in claim 1 or 2, **characterized in that** the angle (α) between the profile sections (9, 16) arranged at an angle to one another is not greater than 150°.

## Revendications

1. Prothèse de disque intervertébral, qui présente deux plaques de recouvrement et une âme de prothèse (5) reliée à l'une des plaques de recouvrement (1) par des profilés complémentaires de liaison (8, 8', 14, 14') en contre-dépouille, **caractérisée en ce que** les profilés de liaison (8, 8', 14, 14') comprennent au moins une paire de parties (9, 16) de profilé disposées symétriquement par rapport à la direction antéropostérieure AP (10) et obliquement l'une par rapport à l'autre.

2. Prothèse de disque intervertébral selon la revendication 1, **caractérisée en ce que** les parties (9, 16) de profilé disposées obliquement l'une par rapport à l'autre s'étendent essentiellement en ligne droite.

3. Prothèse de disque intervertébral selon la revendication 1 ou 2, **caractérisée en ce que** les parties (9, 16) de profilé disposées obliquement l'une par rapport à l'autre sont disposées en position dorsale et sont maintenues en engagement par un dispositif (11, 18) disposé en position ventrale.

4. Prothèse de disque intervertébral selon la revendication 3, **caractérisée en ce que** les parties (9, 16) de profilé disposées dorsalement sont prévues en plus d'une paire de parties (7') de profilé disposée latéralement.

5. Prothèse de disque intervertébral selon la revendication 1 ou 2, **caractérisée en ce que** l'angle (α) entre les parties (9, 16) de profilé disposées obliquement l'une par rapport à l'autre n'est pas plus grand que 150 °.
